# EUROPÄISCHE PATENTANMELDUNG

(11) **EP 3 569 140 A1**
(43) Veröffentlichungstag der Anmeldung: **20.11.2019**
(21) Anmeldenummer: 18172941.9
(22) Anmeldetag: 17.05.2018
(51) Int. Cl.: A61B 5/00

(54) **SYSTEM MIT EINEM IMPLANTAT, INSBESONDERE ZUR PULMONALEN DRUCKMESSUNG, SOWIE MIT EINEM KATHETER ZUM IMPLANTIEREN DES IMPLANTATS**

(71) Anmelder: BIOTRONIK SE & Co. KG, 12359 Berlin (DE)
(72) Erfinder: Degen, Nicolas, 8222 Beringen (CH)
(74) Vertreter: Engelhardt, Björn

(57) **Zusammenfassung**

Die Offenbarung betrifft ein System (1), aufweisend ein Implantat (2) sowie einen Katheter (3) zum Implantieren des Implantats (2) in ein Gefäß (G) eines Patienten, wobei das Implantat (2) ein proximales Verankerungselement (21) aufweist, das von einem proximalen Ende (20a) eines Gehäuses (20) des Implantats (2) absteht, sowie ein distales Verankerungselement (22), das von einem distalen Ende (20b) des Gehäuses (20) absteht, wobei der Katheter (3) einen längserstreckten Schaft (30) aufweist, der ein Hauptlumen (31) zur Aufnahme eines Mandrins (23) aufweist, der in die Verankerungselemente (21, 22) einführbar ist bzw. zum Verankern der Verankerungselemente (21, 22) aus diesen herausziehbar ist.

## Beschreibung

Die Offenbarung betrifft ein System mit einem Implantat, insbesondere zur Druckmessung, sowie mit einem Katheter zum Implantieren des Implantats.

Es ist bekannt, dass Verankerungseinrichtungen von Implantaten, die auf selbstexpandierenden Systemen beruhen, im venösen System (sowie in der Pulmonalarterie) die Tendenz haben, durch das Gefäß zu wachsen. Ein Hauptproblem stellt dabei der durch das Implantat auf die Gefäßwand ausgeübte Druck dar. Dies gilt insbesondere für strebenartige Strukturen von selbstexpandierenden Systemen, die in Kontakt mit der Gefäßwand treten.

Bei selbstexpandierenden Systemen besteht daher grundsätzlich die Schwierigkeit, einen Anpressdruck der besagten Strukturen hinreichend klein zu halten, aber gleichzeitig eine ausreichende Adaption der Konstruktion an die Gefäßwand zu erreichen.

Das Dokument US 7,572,228 B2 offenbart ein Implantat zur Druckmessung in einem Gefäß das wendelförmige Verankerungselemente aufweist.

Hiervon ausgehend liegt der vorliegenden Erfindung die Aufgabe zugrunde, ein System mit einem Implantat und einem Katheter zum Implantieren des Implantats bereitzustellen, das ein präzises Implantieren des Implantats sowie ein sicheres Verankern des Implantats im Gefäß des Patienten erlaubt, so dass insbesondere ein Einwachsen des Implantats in das Gefäß weitestgehend vermieden wird.

Diese Aufgabe wird durch ein System mit den Merkmalen des Anspruchs 1 gelöst. Weitere Ausgestaltungen sind in den Unteransprüchen angegeben und werden nachfolgend detailliert beschrieben.

Gemäß Anspruch 1 wird ein System offenbart, aufweisend ein Implantat sowie einen Katheter zum Implantieren des Implantats in ein Gefäß eines Patienten, wobei das Implantat ein proximales Verankerungselement aufweist, das von einem proximalen Ende eines Gehäuses des Implantats absteht, sowie ein distales Verankerungselement, das von einem distalen Ende des Gehäuses absteht, wobei der Katheter einen längserstreckten Schaft aufweist, der ein Hauptlumen zur Aufnahme eines Mandrins aufweist, der in die Verankerungselemente einführbar ist, wobei die Verankerungselemente durch Herausziehen des Mandrins aus den Verankerungselementen in einen Verankerungszustand überführbar sind, wobei die Verankerungselemente in dem Verankerungszustand jeweils eine vorgegebene Wendelform annehmen.

Es ist vorgesehen, dass der Schaft des Katheters zum Wiedereinführen des Mandrins in die Verankerungselemente vier separate Nebenlumina aufweist, die sich entlang des Hauptlumens erstrecken, wobei ein flexibles sowie längserstrecktes erstes Zugelement in einem ersten Nebenlumen verläuft, an einem distalen Ende des Schafts aus dem Schaft herausgeführt ist, um eine an einem proximalen Ende des proximalen Verankerungselements vorgesehene erste Öse gelegt ist und in einem zweiten Nebenlumen des Schaftes wieder zurück geführt ist, und wobei ein flexibles sowie längserstrecktes zweites Zugelement in einem dritten Nebenlumen verläuft, an dem distalen Ende des Schafts aus dem Schaft herausgeführt ist, um eine an dem proximalen Ende des proximalen Verankerungselementes vorgesehene zweite Öse gelegt ist und in einem vierten Nebenlumen des Schaftes wieder zurückgeführt ist, so dass das distale Ende des Schaftes des Katheters mittels der beiden Zugelemente bei im Verankerungszustand befindlichen Verankerungselementen wieder an das proximale Verankerungselement heranführbar ist.

Es kann vorgesehen sein, dass der Katheter zum Implantat geführt wird, um ein "Herausreißen" der Verankerungselemente zu vermeiden. In dem Fall wird der Schaft in Richtung des verankerten (bewegungslosen) Implantats bewegt. Alternativ kann eine Relativbewegung zwischen dem Schaft und dem Implantat vorgesehen sein, bei welcher beide Komponenten aufeinander zu bewegt werden.

Weiterhin ist gemäß einer Ausführungsform vorgesehen, dass das Implantat, insbesondere das proximale Verankerungselement, zum Implantieren des Implantats so am Schaft des Katheters anordenbar ist, dass das distale Ende des Schaftes ein proximales Ende des proximalen Verankerungselements kontaktiert. Das proximale Verankerungselement und das distale Ende des Schaftes sind also mit anderen Worten auf Stoß angeordnet bzw. anordenbar.

Im Sinne der vorliegenden Erfindung bedeutet "proximal" eine geringere Entfernung einer solchermaßen gekennzeichneten Komponente oder eines solchermaßen gekennzeichneten Ortes entlang des Verlaufs des Katheters, im Vergleich zu einer als "distal" bezeichneten Komponente bzw. eines als "distal" bezeichneten Ortes, die bzw. der eine größere Entfernung zu dem Bediener entlang des Verlaufs des Katheters aufweist.

Das Wendelkonzept der Verankerungselemente zeichnet sich durch eine hohe Adaptionsfähigkeit an komplizierte Anatomien aus, sowie durch eine gut einstellbare Aufstellkraft über einen großen Federbereich. Zudem ist das flexibel einsetzbare Freisetzungsprinzip über ein Mandrin in einer schwer zugänglichen Zielanatomie (z. B. Pulmonalarterie) vorteilhaft.

Bei den Zugelementen kann es sich insbesondere jeweils um einen Faden, ein Seil, eine Faser oder einen Draht handeln.

Weiterhin ist gemäß einer Ausführungsform vorgesehen, dass das jeweilige Zugelement biegeschlaff ausgebildet ist. In diesem Fall kann es sich bei dem jeweiligen Zugelement z. B. um einen Faden, ein Seil, oder eine Faser handeln.

Weiterhin ist gemäß einer Ausführungsform vorgesehen, dass der Katheter ein Führungselement in Form einer Hülse, insbesondere in Form eines Schlauchelements, aufweist, das von dem distalen Ende des Außenschafts absteht. Diese Hülse fungiert insbesondere als Führung für das Implantat bzw. das proximale Verankerungselement, wenn es an den Zugelementen zurück zum Katheter gezogen wird.

Weiterhin ist gemäß einer Ausführungsform vorgesehen, dass sich das Führungselement bzw. Schlauchelement zum distalen Ende des Schaftes hin verjüngt.

Weiterhin ist gemäß einer Ausführungsform vorgesehen, dass das jeweilige Verankerungselement aus einem Metall gebildet ist oder einen metallischen Grundkörper aufweist, der mit einem Kunststoff beschichtet ist (insbesondere mit einem Silikon).

Die vorgegebene Wendelform des Verankerungselements, insbesondere des Grundkörpers kann (bei einem metallischen Verankerungselement bzw. Grundkörper) durch Formglühen des jeweiligen Verankerungselementes bzw. Grundkörpers erzeugt werden.

Weiterhin ist gemäß einer Ausführungsform vorgesehen, dass die Verankerungselemente bei in die Verankerungselemente eingeführtem Mandrin einen geringeren Außendurchmesser aufweisen als im Verankerungszustand.

Die beiden Verankerungselemente sind insbesondere elastisch deformierbar ausgebildet, so dass sie bei eingeführtem Mandrin versuchen, die jeweils vorgegebene Wendelform anzunehmen, derart, dass beim Herausziehen des Mandrins aus dem jeweiligen Verankerungselement das jeweilige Verankerungselement in die vorgegebene Wendelform übergeht.

Weiterhin weist das jeweilige Verankerungselement bei einem in das jeweilige Verankerungselement eingeführten Mandrin zumindest einen Außendurchmesser auf, der kleiner ist als der Außendurchmesser des jeweiligen Verankerungselements im Verankerungszustand sowie kleiner als der Innendurchmesser des Hauptlumens, so dass das Implantat im Hauptlumen verschieblich anordenbar ist. Hierbei weist das jeweilige Verankerungselement insbesondere auch eine größere Längserstreckung als im Verankerungszustand auf.

Insbesondere können die Verankerungselemente im implantierbaren Zustand des Implantats auch eine linear erstreckte Form, d. h., eine vollständig gestreckte Form aufweisen.

Der Außendurchmesser des jeweiligen Verankerungselementes meint im Rahmen der vorliegenden Erfindung den durch die Wendelform bedingten Außendurchmesser. Erstreckt sich das betrachtete Verankerungselement vollkommen linear, ist der Außendurchmesser des betreffenden Verankerungselements durch den Außendurchmesser des nunmehr zylindrischen Verankerungselementes gegeben.

Gemäß einer Ausführungsform ist weiterhin vorgesehen, dass das Implantat dazu konfiguriert ist, in der Pulmonalarterie des Patienten implantiert zu werden.

Weiterhin ist gemäß einer Ausführungsform vorgesehen, dass die Verankerungselemente dazu konfiguriert sind, in der Pulmonalarterie des Patienten implantiert bzw. verankert zu werden.

Weiterhin ist gemäß einer Ausführungsform vorgesehen, dass der Mandrin an dem Gehäuse des Implantats vorbeigeführt ist, wenn der Mandrin in die beiden Verankerungselemente eingeführt ist.

Weiterhin ist gemäß einer Ausführungsform vorgesehen, dass der Mandrin so in die Verankerungselemente einführbar ist, dass er beim Herausziehen zuerst das distale Verankerungselement und sodann das proximale Verankerungselement freigibt bzw. in den jeweiligen Verankerungszustand überführt, in dem das jeweilige Verankerungselement seine vorgegebene Wendelform einnimmt.

Weiterhin ist gemäß einer Ausführungsform vorgesehen, dass sich die vorgegebene Wendelform des proximalen Verankerungselements von der vorgegebenen Wendelform des distalen Verankerungselementes unterscheidet, insbesondere hinsichtlich des Außendurchmessers und oder hinsichtlich der Steigung der jeweiligen Wendelform.

Weiterhin ist gemäß einer Ausführungsform vorgesehen, dass die vorgegebene Wendelform des distalen Verankerungselements, das insbesondere auf der herzabgewandten Seite liegt, eine größere Steigung aufweist als die vorgegebene Wendelform des proximalen Verankerungselements.

Beide Wendeln können also leicht an verschiedene Anatomien in Bezug auf Steigung, Außendurchmesser und insbesondere Härte angepasst werden. Ebenso kann die jeweilige Wendel asymmetrisch ausgebildet sein. Insbesondere kann die Steigung des Wendels variieren.

Weiterhin ist gemäß einer Ausführungsform vorgesehen, dass der Schaft eine Wandung aufweist, die das Hauptlumen umgibt, wobei die Nebenlumina in der Wandung angeordnet sind. Die Wandung kann insbesondere mehrere Schichten aus insbesondere verschiedenen Materialien aufweisen. Als Materialien für die Wandung kommen z. B. Silikone, thermoplastische Elastomere, insbesondere TPE-A (z. B. Pebax), etc. in Frage.

Weiterhin ist gemäß einer Ausführungsform vorgesehen, dass das erste und das zweite Nebenlumen benachbart zueinander in einem ersten Abschnitt der Wandung angeordnet sind und somit ein Nebenlumenpaar bilden, und wobei das dritte und das vierte Nebenlumen benachbart zueinander in einem zweiten Abschnitt der Wandung angeordnet sind und somit ein weiteres Nebenlumenpaar bilden, wobei der zweite Abschnitt quer zu einer Längsachse des Schafts dem ersten Abschnitt gegenüberliegt, so dass das Hauptlumen insbesondere zwischen den beiden Nebenlumenpaaren bzw. zwischen den beiden Abschnitten der Wandung angeordnet ist.

Weiterhin ist gemäß einer Ausführungsform vorgesehen, dass das Implantat einen in dem Gehäuse angeordneten Drucksensor zum Messen eines Drucks in dem Gefäß des Patienten, insbesondere in der Pulmonalarterie des Patienten, aufweist.

Die hier offenbarte Lösung zeichnet sich durch ein Verankerungssystem aus, das im Hinblick auf komplizierte Anatomien hoch adaptionsfähig ist. Die Verankerungselemente zeichnen sich insbesondere durch einen großen Federbereich aus, wobei im Vergleich zu selbstexpandierenden Systemen geringe Anpressdrücke vorliegen, was das Risiko eines Einwachsens des Implantats in die Gefäßwand mindert.

Weiteerhin zeichnet sich der Katheter bzw. Außenschaft des Systems durch eine gute Manövrierbarkeit, eine geringe Profilgröße sowie durch ein kraftfreies Lösen und eine kraftfreie Wiederaufnahme des Implantats aus.

Weitere Merkmale und Ausführungsformen werden nachfolgend anhand der Figuren erläutert. Es zeigen:
- Fig. 1: eine Darstellung eines Herzens eines Patienten sowie insbesondere die Zielanatomie des zu implantierenden Implantats (z. B. Pulmonalarterie);
- Fig. 2: eine schematische Darstellung einer Ausführungsform eines Implantats mit einem distalen und einem proximalen Verankerungselement in einem implantierten Zustand;
- Fig. 3: eine Schnittdarstellung eines Schafts eines Katheters eines Systems;
- Fig. 4: ein proximales Ende des proximalen Verankerungselements mit Ösen für die Zugelemente des Katheters;
- Fig. 5: einen distalen Endabschnitt des Schaftes des Katheters eines Systems mit einem Führungselement zum Führen des proximalen Ankerelements bzw. Implantats zum Wiedereinführen des Mandrins in die Verankerungselemente;
- Fig. 6: eine weitere Darstellung des distalen Endabschnitts mit dem Führungselement, wobei die Lage des Hauptlumens und der Nebenlumina am distalen Ende des Schaftes erkennbar ist; und
- Fig. 7: eine weitere Darstellung des distalen Endabschnitts des Schaftes des Katheters eines Systems beim Fixieren des Implantats bezüglich des Außenschaftes mittels der Zugelemente.

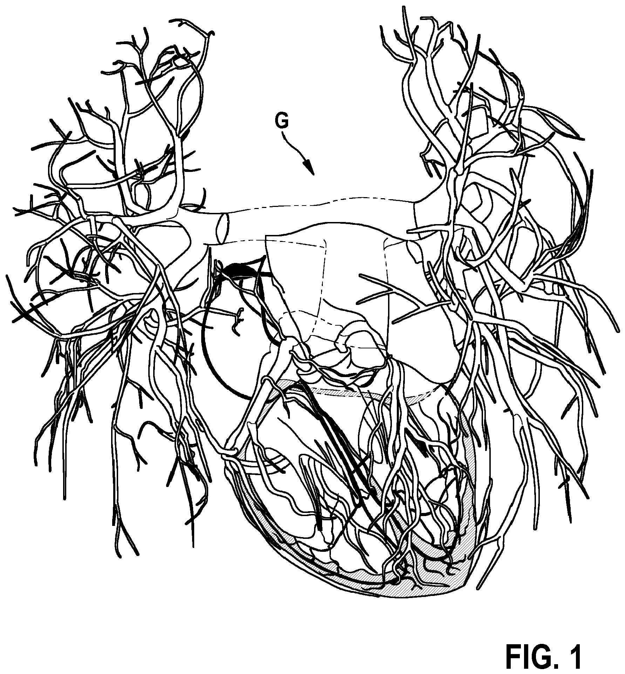

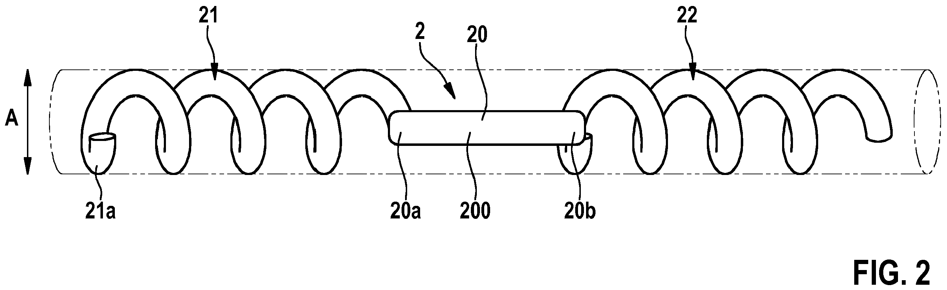

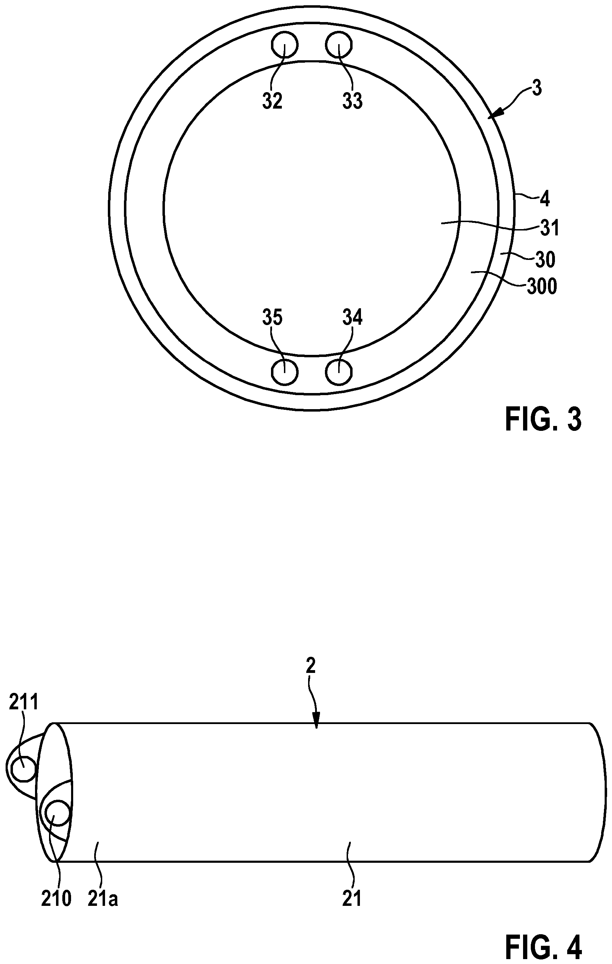

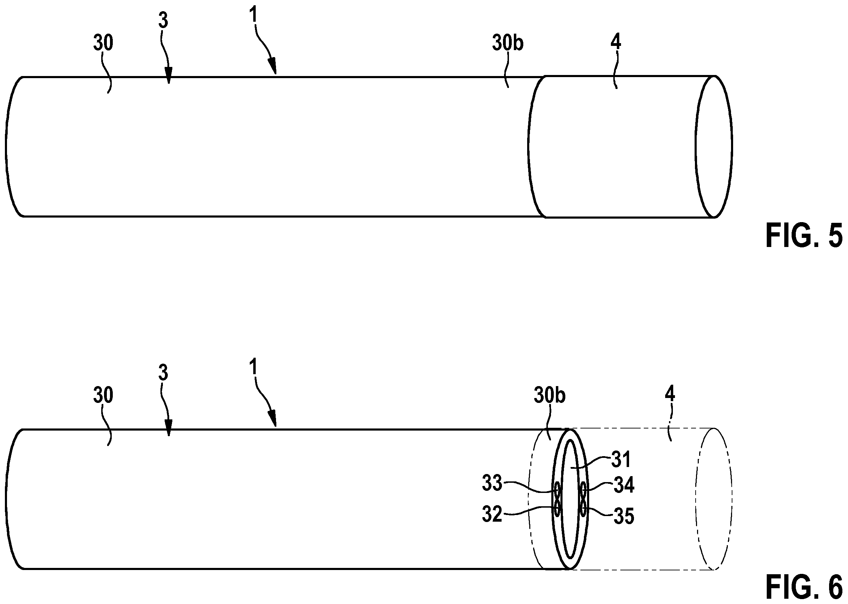

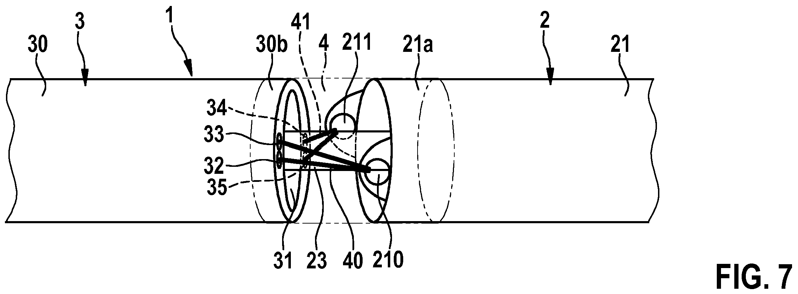

Fig. 2 zeigt im Zusammenhang mit den Figuren 3 bis 7 eine Ausführungsform eines Systems 1, aufweisend ein Implantat 2, das hier z. B. einen in einem Gehäuse 20 angeordneten Sensor 200 zur Druckmessung des Blutes in einem Gefäß G des Patienten aufweist, in das das Implantat 2 zu implantieren ist, sowie einen Katheter 3 zum Implantieren des Implantats 2 in das Gefäß G, bei dem es sich insbesondere gemäß Figur 1 um die Pulmonalarterie des Patienten handelt. Das Implantat 2 weist gemäß Figur 2 ein proximales Verankerungselement 21 auf, das von einem proximalen Ende 20a des Gehäuses 20 absteht, sowie ein distales Verankerungselement 22, das von einem distalen Ende 20b des Gehäuses 20 absteht, und zwar insbesondere in eine entgegengesetzte Richtung. Der Katheter 3 weist einen längserstreckten Schaft 30 auf, der ein Hauptlumen 31 zur Aufnahme eines Mandrins 23 aufweist, der in der Figur 7 exemplarisch gezeigt ist, wobei das Implantat 2 bei einem in die Verankerungselemente 21, 22 eingeführten Mandrin 23 in dem Gefäß G verschieblich ist, und wobei die Verankerungselemente 21, 22, wie es in der Fig. 2 angedeutet ist, durch Herausziehen des Mandrins 23 aus den Verankerungselementen 21, 22 in einen Verankerungszustand überführbar sind, wobei die Verankerungselemente 21, 22 in dem Verankerungszustand jeweils eine vorgegebene Wendelform annehmen bzw. ein Wendel 21, 22 ausbilden.

Es ist vorgesehen, dass der Schaft 30 des Katheters 3 zum Wiedereinführen des Mandrins 23 in die Verankerungselemente 21, 22 ein erstes Nebenlumen 32, ein zweites Nebenlumen 33, ein drittes Nebenlumen 34 und ein viertes Nebenlumen 35 aufweist (vgl. insbesondere Fig. 7), wobei ein flexibles erstes Zugelement 40 in dem ersten Nebenlumen 32 verläuft, an einem distalen Ende 30b des Schafts 30 aus dem Schaft 30 herausgeführt ist, um eine an einem proximalen Ende 21a des proximalen Verankerungselements 21 vorgesehene erste Öse 210 gelegt ist und in dem zweiten Nebenlumen 33 des Schaftes 30 wieder zurück geführt ist, und wobei ein flexibles zweites Zugelement 41 in dem dritten Nebenlumen 34 verläuft, an dem distalen Ende 30b des Schafts 30 aus dem Schaft 30 herausgeführt ist, um eine an dem proximalen Ende 21a des proximalen Verankerungselementes 21 vorgesehene zweite Öse 211 gelegt ist und in dem vierten Nebenlumen 35 des Schaftes 30 wieder zurückgeführt ist, so dass das distale Ende 30b des Schaftes 30 bei Bedarf wieder an das proximale Verankerungselement 21 im verankerten Zustand des Implantats 2 mittels der beiden Zugelemente 40, 41 heranführbar ist.

Freigesetzt bzw. verankert wird das anfänglich insbesondere auf Stoß am distalen Ende 30b des Schafts 30 angeordnete Implantat 2 beim Implantieren, indem der Mandrin 23 zurückgezogen wird. Der Mandrin 23 wird vorzugsweise seitlich an dem Implantat 2 vorbeigeführt und gibt zuerst die katheterabgewandte Seite, d. h., das distale Verankerungselement 22 frei und zuletzt die Seite, die am Katheter 3 liegt, d. h., das proximale Verankerungselement 21.

Grundsätzliche Probleme solcher mandrinbasierten Systeme bestehen darin, dass ein in dieser Form freigesetztes Implantat nicht mehr ohne weiteres zurückgezogen werden kann, wenn erst einmal der Mandrin 23 zurückgezogen worden ist.

Um das Wiedereinfangen des Implantats 2 nach einem vollständigen Freisetzen in dem Gefäß G zu ermöglichen, muss der Mandrin 23 wieder in die Verankerungselemente 21, 22 eingeführt werden. Dies wird dadurch ermöglicht, indem das Implantat 2, insbesondere das proximale Verankerungselement 21 durch die Zugelemente 40, 41 an das distale Ende 30b des Schafts 30 herangezogen werden kann bzw. das Schaftende 30b an das proximale Verankerungselement 21 heranführbar ist, wobei das Implantat 2 solchermaßen am Schaft 30 positionierbar bzw. festlegbar ist, vorzugsweise auf Stoß mit dem distalen Ende 30b, dass der Mandrin 23 wieder in die Verankerungselemente 21, 22 einführbar ist.

Wie bereits eingangs beschrieben, werden die beiden Zugelemente 40, 41, die insbesondere als Fäden ausgebildet sein können, in dem Katheter 3 geführt und verlassen am proximalen Ende des Schaftes 30 (außerhalb des Patienten) den Katheter 3. Die Schlaufen der Fäden bzw. Zugelemente 40, 41 gehen durch die Ösen 210, 211 des proximalen Verankerungselementes 21. Weiterhin ist am distalen Ende 30b des Schaftes 30 ein Führungselement 4 in Form einer Hülse (z. B. in Form eines festen Schlauchabschnitts) 4 angebracht. Dieses Führungselement 4 kann auch zum distalen Ende 30b des Schaftes 30 des Katheters 3 hin im Querschnitt verjüngt ausgebildet sein und fungiert als Führung für das Implantat 2 bzw. das proximale Verankerungselement 21 wenn es an den Fäden/Zugelementen 40, 41 zurück auf das Katheter 3 gezogen wird.

Ist der Arzt bzw. Bediener des Katheters 3 mit der Position des Implantats 2 noch nicht zufrieden, können das Implantat 2 und der Schaft 30 des Katheters 3 (nach einem vollständigem Freisetzen bzw. Verankern durch Zurückziehen des Mandrins 23) an den Fäden bzw. Zugelementen 40, 41 am proximalen Ende des proximalen Verankerungselements 21 wieder zusammen gebracht werden, wobei durch die solchermaßen hergestellte geometrische Fixierung bzw. bauartbedingte Positionierung des Implantats 2 am distalen Ende 30b des Schaftes 30 der Mandrin 23 wieder in das Implantat 2 bzw. die Verankerungselemente 21, 22 einführbar ist, wodurch in der Folge alle Teile der Wendel 21, 22 wieder begradigt werden können, so dass eine neue Positionierung des Implantats 2 im Gefäß G erfolgen kann.

Die besagte geometrische Fixierung wird insbesondere durch das Führungselement 4 verbessert, das bei der Annäherung zwischen dem Implantat 2 und dem Schaftende 30b für eine Fixierung sorgt, derart, dass der Mandrin 23 wieder durch den Katheter 3 in das Implantat 2 gesteckt werden kann.

Nachdem der Arzt bzw. Bediener eine finale Position gefunden hat und die Verankerungselemente 21, 22 durch Herausziehen des Mandrins 23 im Gefäß G verankert sind, können die beiden Fäden/Zugelemente 40, 41 aus den Nebenlumina 32, 33, 34, 35 des Katheters 3 vollständig herausgezogen werden, womit das Implantat 2 restlos vom Katheter 3 abgekoppelt wird.

## Patentansprüche

1. System (1), aufweisend ein Implantat (2) sowie einen Katheter (3) zum Implantieren des Implantats (2) in ein Gefäß (G) eines Patienten, wobei das Implantat (2) ein proximales Verankerungselement (21) aufweist, das von einem proximalen Ende (20a) eines Gehäuses (20) des Implantats (2) absteht, sowie ein distales Verankerungselement (22), das von einem distalen Ende (20b) des Gehäuses (20) absteht, wobei der Katheter (3) einen längserstreckten Schaft (30) aufweist, der ein Hauptlumen (31) aufweist, und wobei das System (1) weiterhin einen in dem Hauptlumen (31) führbaren und in die Verankerungselemente (21, 22) einführbaren Mandrin (23) aufweist, wobei die Verankerungselemente (21, 22) durch Herausziehen des Mandrins (23) aus den Verankerungselementen (21, 22) in einen Verankerungszustand überführbar sind, wobei die Verankerungselemente (21, 22) in dem Verankerungszustand jeweils eine vorgegebene Wendelform annehmen, wobei der Schaft (30) des Katheters (3) zum Wiedereinführen des Mandrins (23) in die Verankerungselemente (21, 22) ein erstes Nebenlumen (32), ein zweites Nebenlumen (33), ein drittes Nebenlumen (34) und ein viertes Nebenlumen (35) aufweist, wobei ein flexibles erstes Zugelement (40) in dem ersten Nebenlumen (32) verläuft, an einem distalen Ende (30b) des Schafts (30) aus dem Schaft (30) herausgeführt ist, um eine an einem proximalen Ende (21a) des proximalen Verankerungselements (21) vorgesehene erste Öse (210) gelegt ist und in dem zweiten Nebenlumen (33) des Schaftes (30) wieder zurück geführt ist, und wobei ein flexibles zweites Zugelement (41) in dem dritten Nebenlumen (34) verläuft, an dem distalen Ende (30b) des Schafts (30) aus dem Schaft (30) herausgeführt ist, um eine an dem proximalen Ende (21a) des proximalen Verankerungselementes (21) vorgesehene zweite Öse (211) gelegt ist und in dem vierten Nebenlumen (35) des Schaftes (30) wieder zurückgeführt ist, so dass das distale Ende (30b) des Schafts (30) mittels der beiden Zugelemente (40, 41) bei im Verankerungszustand befindlichen Verankerungselementen (21, 22) wieder an das proximale Verankerungselement (21) heranführbar ist.

2. System nach Anspruch 1, wobei das jeweilige Zugelement (40, 41) biegeschlaff ausgebildet ist.

3. System nach Anspruch 1 oder 2, wobei der Katheter (3) ein Führungselement (4) in Form einer Hülse aufweist, die von dem distalen Ende (30b) des Schafts (30) absteht.

4. System nach Anspruch 3, wobei sich das Führungselement (4) zum distalen Ende (30b) des Schaftes (30) hin verjüngt.

5. System nach einem der vorhergehenden Ansprüche, wobei das jeweilige Verankerungselement (21, 22) einen metallischen Grundkörper aufweist, der mit einem Kunststoffbeschichtet ist.

6. System nach einem der vorhergehenden Ansprüche, wobei die Verankerungselemente (21, 22) bei in die Verankerungselemente (21, 22) eingeführtem Mandrin (23) einen geringeren Außendurchmesser (A) aufweisen als im Verankerungszustand.

7. System nach einem der vorhergehenden Ansprüche, wobei die Verankerungselemente (21, 22) dazu konfiguriert sind, in der Pulmonalarterie (G) verankert zu werden.

8. System nach einem der vorhergehenden Ansprüche, wobei der Mandrin (23) an dem Gehäuse (20) des Implantats (2) vorbeigeführt ist, wenn der Mandrin (23) in die beiden Verankerungselemente (21, 22) eingeführt ist.

9. System nach einem der vorhergehenden Ansprüche, wobei der Mandrin (23) so in die Verankerungselemente (21, 22) einführbar ist, dass er beim Herausziehen zuerst das distale Verankerungselement (22) und sodann das proximale Verankerungselement (21) freigibt.

10. System nach einem der vorhergehenden Ansprüche, wobei sich die vorgegebene Wendelform des proximalen Verankerungselements (21) von der vorgegebenen Wendelform des distalen Verankerungselementes (22) unterscheidet.

11. System nach einem der vorhergehenden Ansprüche, wobei die vorgegebene Wendelform des distalen Verankerungselements (22) eine größere Steigung aufweist als die vorgegebene Wendelform des proximalen Verankerungselements (21).

12. System nach einem der vorhergehenden Ansprüche, wobei der Schaft (30) eine Wandung (300) aufweist, die das Hauptlumen (31) umgibt, wobei die Nebenlumina (32, 33, 34, 35) in der Wandung (300) angeordnet sind.

13. System nach einem der vorhergehenden Ansprüche, wobei das Implantat (2) einen in dem Gehäuse (20) angeordneten Drucksensor (200) zum Messen eines Drucks in dem Gefäß (G) des Patienten aufweist.
